# FASCICULE DE BREVET EUROPEEN

(11) **EP 1 212 060 B1**
(45) Date de publication et mention de la délivrance du brevet: **13.04.2005**
(21) Numéro de dépôt: 00960782.1
(22) Date de dépôt: 01.09.2000
(51) Int. Cl.: A61K 31/465, A61K 31/195, A61P 25/28, A61P 25/16

(54) **UTILISATION DE LA NICOTINE OU DE SES DERIVES ET DE L-DOPA DANS UN MEDICAMENT POUR LE TRAITEMENT DES MALADIES NEUROLOGIQUES, NOTAMMENT LA MALADIE DE PARKINSON**
VERWENDUNG VON NIKOTIN ODER DERIVATEN UND L-DOPA ZUR BEHANDLUNG VON NEUROLOGISCHEN KRANKHEITEN, INSBESONDER MORBUS PARKINSON
USE OF NICOTINE OR DERIVATIVES THEREOF AND L-DOPA IN A MEDICINE FOR TREATING NEUROLOGICAL DISEASES, IN PARTICULAR PARKINSON DISEASE

(30) Priorité: 02.09.1999 FR 9911029
(43) Date de publication de la demande: 12.06.2002
(73) Titulaire: ASSISTANCE PUBLIQUE, HOPITAUX DE PARIS, 75100 Paris (FR)
(72) Inventeur: VILLAFANE, Gabriel, F-75013 Paris (FR); CESARO, Pierre, F-94410 Saint-Maurice (FR)
(74) Mandataire: Desaix, Anne
(86) Numéro de dépôt international: PCT/FR2000/002428
(87) Numéro de publication internationale: WO 2001/015696

(56) Documents cités:
- US-A- 5 232 933
- E. F. DOMINO ET AL: "Nicotine Alone and in Combination with L-Dopa Methyl Ester or the D2 Agonist N-0923 in MPTP-Induced Chronic Hemiparkinsonian Monkeys" EXPERIMENTAL NEUROLOGY, vol. 158, août 1999 (1999-08), pages 414-421, XP000910817
- BALFOUR D J K ET AL: "PHARMACOLOGY OF NICOTINE AND ITS THERAPEUTIC USE IN SMOKING CESSATION AND NEURODEGENERATIVE DISORDERS" PHARMACOLOGY AND THERAPEUTICS,GB,ELSEVIER, vol. 72, no. 1, 1996, pages 51-58, XP000900095 ISSN: 0163-7258 cité dans la demande

## Description

La présente invention porte sur l'utilisation de dérivés nicotiniques dans la fabrication de médicaments pour traiter la maladie de Parkinson et les atrophies multi-systématisées.

La maladie de Parkinson et les maladies associées n'ont pas de définition incontestée, ce qui est lié au fait que l'on en ignore encore la cause.

Le diagnostic d'une maladie de Parkinson est réalisé par un faisceau de syndromes cliniques qui se caractérise par des signes moteurs (tremblement de repos, rigidité, hypokinésie et instabilité posturale) et des déficits de nature neuropsychologique pouvant même affecter certaines fonctions cognitives.

D'autres affections dites atrophies multisystémiques peuvent, parfois, pendant plusieurs années, évoluer cliniquement comme une maladie de Parkinson. Il s'agit en particulier de l'atrophie strio-nigrique, l'atrophie ponto-cérébelleuse, ou le syndrome de Shy-Drager.

Les récepteurs jouent un rôle considérable dans la maladie de Parkinson car ils sont le site d'action de la dopamine libérée par l'élément pré-synaptique.

Les récepteurs D1 sont préférentiellement stimulés par la dopamine. Ils sont situés sur la membrane post-synaptique et ils sont couplés à l'activité adénylate-cyclase. Ils sont localisés dans le striatum, le noyau accumbens, le tubercule olfactif.

Les récepteurs D2 sont stimulés préférentiellement par certains agonistes dopaminergiques comme la bromocryptine et le pyribédil.

Nombre d'études, tant chez l'animal que chez l'homme, ont démontré que ces troubles sont en grande partie associés à une dégénérescence chronique des neurones dopaminergiques du système nigrostriatal. Le traitement actuellement existant, et qui reste le traitement de référence, est un traitement avec la L-DOPA le cas échéant accompagné d'agonistes des récepteurs D2, tels ceux cités ci-dessus. Néanmoins, ce type de traitement présente des effets secondaires à moyen et long terme tels des dyskinésies.

L'inconvénient induit par ces effets indésirables a encouragé la recherche d'autres thérapeutiques notamment chirurgicales comme la greffe de neurones dopaminergiques (Olanow C.W. et al (1996) Trends Neurosciences 19: 102-109).

L'objectif poursuivi par ce type de greffe est le développement de l'innervation dopaminergique dans le striatum qui vient remplacer celle défaillante du patient parkinsonien. Malgré tout, cette technique est particulièrement lourde, et est, comme toute greffe, dépendante de la disponibilité de neurones donneurs qui sont généralement obtenus par prélèvement chez un foetus au cours d'une interruption volontaire de grossesse. Il semblerait néanmoins que si cette greffe, quand elle est pratiquée de façon bilatérale dans le putamen, peut améliorer la bradykinésie, l'akinésie et la rigidité chez le patient parkinsonien, elle n'a pas d'effets sûrs concernant la bradyphrénie et les autres fonctions cognitives. Ce type de traitement, outre les inconvénients liés à la greffe cités plus haut, ne résout pas le problème du traitement de la maladie de Parkinson et de ses différents symptômes associés notamment sur le plan neuropsychologique (J. Doyen et al., M Medecine et Science (1999), 5: XIX-XXIII).

Il a par ailleurs été observé que l'incidence de la maladie de Parkinson est significativement plus faible chez les fumeurs que chez les non fumeurs. Il a été suggéré que la nicotine possède la propriété d'activer les récepteurs cholinergiques nicotiniques par administration aiguë et de provoquer une augmentation du nombre de tels récepteurs par administration chronique de nicotine chez l'animal (D.J.K. Balfour et al., Pharmacology and Therapeutics (1996) 72, fasc 1: 51-81). Néanmoins, tous les essais de traitement à la nicotine réalisés ne l'ont jamais été au cours d'un traitement à long terme, sans interruption. Par long terme, on entend une période supérieure à trois mois. En outre, la mise au point d'un traitement associant la nicotine ou ses dérivés, la L-DOPA et les agonistes des récepteurs D2 n'a jamais été recherchée.

La mise au point d'un médicament et plus généralement d'un traitement permettant de restaurer les fonctionnalités des récepteurs dopaminergiques D1 et D2 reste un problème majeur dans le domaine des maladies neurodégénératives.

Par nicotine, on entend tant la nicotine que ses différents dérivés à partir du moment où ils sont susceptibles de se coupler aux récepteurs nicotinergiques et d'être des agonistes de ces récepteurs. A titre d'exemple, on peut citer la catioresine carboxylate de nicotine.

Les dérivés de la nicotine ont été décrits comme utilisables dans des traitements de la maladie de Parkinson. On peut citer, à ce titre, les brevets US 5,232,933 et 5,242,935. Néanmoins, dans ces brevets, la durée d'administration n'est pas donnée et les expérimentations sont effectuées sur des souris ou des rats. En outre, les symptômes classiques, que ce soit les symptômes physiques ou neuro-psychologiques ne sont pas observés. Enfin, rien n'est dit sur un traitement simultané ou séparé avec la L-DOPA et les agonistes des récepteurs D2.

Une étude réalisée par A.M. Janson et al. (1994), 655: 25-32 fait état de résultats suite également à un traitement chronique chez le rat. Le traitement dit à long terme est également dans ce cas-là de 14 jours, ce qui n'est pas comparable au traitement par le médicament selon l'invention.

La présente invention porte sur l'utilisation dans la fabrication d'un médicament pour administration de façon continue ou progressive de 0,2 à 5 mg par jour et par kilo de poids corporel chez l'homme de nicotine ou un dérivé de celle-ci, ledit médicament étant administré de façon simultanée avec la L-DOPA à des doses au moins 30 % inférieures aux doses efficaces quand elle est administrée seule. Une dose préférée se situe entre 0,2 et 3 mg par kilo et par 24 heures.

Un tel médicament est destiné au traitement des maladies neuro-dégénératives, et notamment de la maladie de Parkinson et du syndrome de Gilles de La Tourette.

De façon préférée, les doses de L-DOPA administrées simultanément avec les médicaments selon l'invention sont au moins 50 % inférieures aux doses efficaces quand elle est administrée seule.

Le traitement normal utilisé en routine pour traiter la maladie de Parkinson peut être constitué d'une administration quotidienne de 200 mg par jour à 1 500 mg par jour et les doses des agonistes dopaminergiques sont tout à fait aléatoires et dépendent de chaque agoniste dopaminergique.

Compte tenu des effets secondaires de traitement de la L-DOPA à moyen terme, la diminution de la dose du L-DOPA de 30 voire de 50 ou 60 % par rapport à cette dose représente des avantages certains.

Le médicament selon l'invention est administré selon un protocole qui implique l'augmentation progressive de doses cumulatives de nicotine ou de ses dérivés, et ce pendant trois mois consécutifs, suivi par des doses stabilisées après trois mois. L'augmentation de la dose de nicotine pendant les trois à quatre premiers mois est accompagnée d'une diminution concomitante de la dose de L-DOPA selon l'amélioration des syndromes parkinsoniens constatée.

A titre d'exemple, l'administration est de 0,2 mg par kilo par 24 heures pendant trois à quatre mois, puis 1,4 à 1,5 mg par kilo par 24 heures à partir du troisième ou quatrième mois, puis, en fonction de l'amélioration des symptômes et du besoin thérapeutique du patient, la dose est ajustée entre 2 et 3 mg par kilo par 24 heures.

La présente invention porte également sur une méthode de traitement des maladies neuro-dégénératives et notamment la maladie de Parkinson ou du syndrome de Gilles de la Tourette, comprenant une administration à long terme de 0,2 à 5 mg par jour et par kilo de poids corporel chez l'homme de nicotine ou un dérivé de celle-ci ; cette administration est administrée de façon simultanée à des doses de L-DOPA non actives lorsque cette dernière est administrée seule. Il s'agit d'un traitement à long terme, c'est-à-dire d'une durée minimale de quatre mois et de préférence de six mois, ce qui permet aux symptômes physiques et neuro-psychologiques de diminuer voire de disparaître, et ce de façon stable. Les symptômes étudiés sont pour les symptômes physiques : le tremblement, la rigidité axiale, la marche, la parole. Les symptômes neuro-psychologiques sont la mémoire et l'activité sexuelle.

L'évaluation clinique de l'effet du médicament selon l'invention et du procédé de traitement a été réalisée en utilisant les tests neurologiques classiques UPDRS I, II et III. Ces tests permettent de mesurer tant les effets neuro-psychologiques que les effets physiques.

Le test UPDRS I mesure sur une échelle de 0 à 4 à l'état mental compartemental ou thymique du patient, prenant comme critères notamment l'affaiblissement intellectuel, les troubles de la pensée, la dépression et la motivation / initiative.

Le test UPDRS II mesure une série de tests physiques sensitifs ou moteurs, également sur une échelle allant de 0 à 4.

Le test UPDRS III mesure l'activité motrice du patient, sur une échelle allant de 0 à 4.

Zéro indique un état normal et quatre indique une perturbation maximale.

Il est essentiel qu'un traitement avec les médicaments selon l'invention soit un traitement à long terme ; ainsi, les différents modes d'administration de ce médicament doivent être compatibles avec ce type de traitement. En particulier, le médicament selon l'invention peut être administré par voie sous-cutanée en patch de libération rapide et lente en continu à l'aide d'une pompe de type extracorporelle. Un cathéter sous-cutané permet d'administrer en permanence la dose de nicotine requise, et adaptable par un régulateur de vitesse alimenté par des piles.

Le médicament peut également être administré par voie orale, dans une forme galénique comprenant également la L-DOPA et/ou les agonistes dopaminergiques.

Le médicament selon l'invention permet la multiplication, la simulation et l'augmentation des récepteurs nicotinergiques ainsi que des récepteurs D1 et D2 pré-synaptiques et post-synaptiques dans la zone niger-striatum, ce qui peut être mis en évidence par un examen PET (tomographie par émission de positions), en utilisant la F18-DOPA et la raclopride respectivement, selon la technique décrite dans Médecine/Sciences (1999), 15: 490-495.

L'exemple de réalisation ci-après montre les avantages inattendus du médicament selon l'invention, administré concomitamment avec la L-DOPA et du procédé de traitement de la maladie de Parkinson et de ses maladies associées.

### Figures :

La figure 1 représente les doses respectives de L-DOPA, de bromocryptine et de nicotine administrées au cours du temps, exprimées en mg.
La figure 2 représente les résultats des tests UPDRS en l'absence de L-DOPA (off) ou en présence de L-DOPA (on). La dose de nicotine est indiquée par les barres blanches de l'histogramme.

### Exemple 1 :

Un patient âgé de 57 ans était traité depuis 10 ans avec 800 mg par jour de L-DOPA, complétés par 30 mg par jour de bromocryptine.

Pendant une durée de six mois, des patchs de nicotine ont été administrés au patient, d'une part en utilisant des doses croissantes pendant les trois premiers mois pour arriver à une dose de 102 mg par jour et, d'autre part en combinant deux types de patch, des patch sous forme de libération retard (patch P) et des formes à libération rapide (patch R). Chaque patch P contient 21 mg de nicotine et chaque patch R contient 15 mg de nicotine. La cinétique d'administration pendant les douze premiers mois est représentée dans le tableau I ci-dessous.

**Tableau I :**

| **Mois** | **Patch P** | **Patch R** | **Doses R** | **Total doses/jour** |
|---|---|---|---|---|
| 1 | 21 mg de nicotine | | | 21 mg |
| 2 | 42 mg de nicotine | | | 42 mg |
| 3 | 63 mg de nicotine | 2 doses | 30 mg (LR) (2 x 15 mg) | 93 mg |
| 4 | 42 mg de nicotine | 4 doses | 60 mg (4 x 15 mg) | 102 mg |
| 5 | 42 mg de nicotine | 4 doses | 60 mg (4 x 15 mg) | 102 mg |
| 6 | 42 mg de nicotine | 4 doses | 60 mg (4 x 15 mg) | 102 mg |
| 7-12 | 42 mg de nicotine | 4 doses | 60 mg (4 x 15 mg) | 102 mg |

Globalement, la dose quotidienne est de 21 mg le premier mois pour se stabiliser à 102 mg à partir du quatrième mois décomposée en 42 mg à libération contrôlée et 60 mg à libération rapide.

### Doses de L-DOPA et bromocryptine :

De façon concomitante, la L-DOPA est administrée à une dose décroissante au cours du temps ; la dose de bromocryptine, en revanche est inchangée et reste pendant toute la durée du traitement à 30 mg par jour, et ce selon le tableau II ci-dessous.

**Tableau II :**

| **MOIS** | **L-DOPA** | **Nicotine** | **Bromocryptine** |
|---|---|---|---|
| 1 | 800 | 0 | 30 |
| 2 | 800 | 21 | 30 |
| 3 | 800 | 42 | 30 |
| 4 | 600 | 93 | 30 |
| 5 | 400 | 102 | 30 |
| 6 | 300 | 102 | 30 |
| 7 | 200 | 102 | 30 |
| 8 | 200 | 102 | 30 |

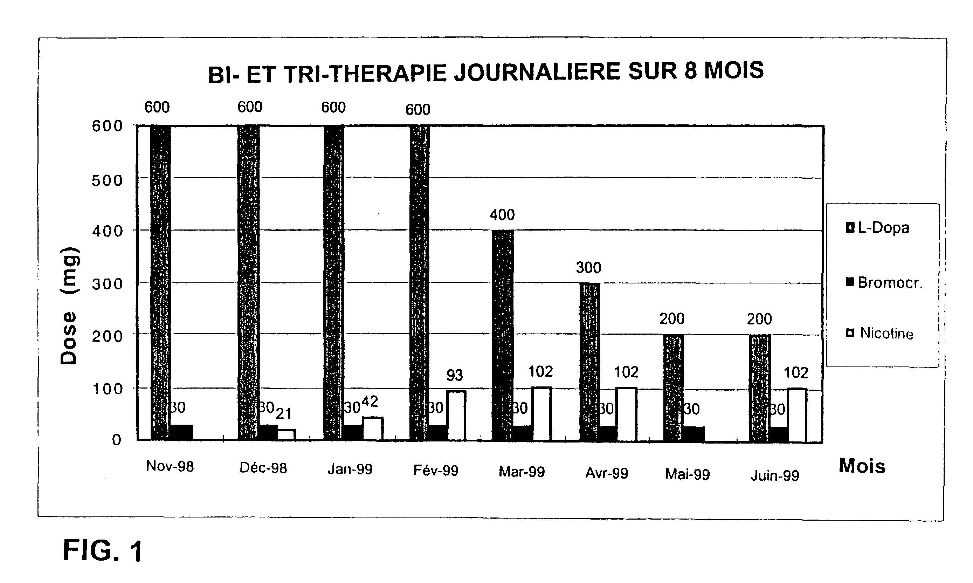

La figure 1 représente une vue synthétique du traitement au cours du temps. L'arrêt de l'administration de nicotine au huitième mois n'a pas d'effet symptomatique.

### Exemple 2 : effet des médicaments selon l'invention et du traitement nicotine sur les manifestations cliniques de la maladie de Parkinson et des atrophies multisystématisées :

L'évaluation clinique est basée sur le test à la L-DOPA et est réalisée de la façon suivante :

Le test UPDRS utilisé est décrit ci-dessous.

Il consiste à sevrer le patient de L Dopathérapie à 0 dose entre 12 et 24 heures avant ce test.

Le lendemain, toutes les demi-heures, on mesure l'état et les changements cliniques du patient pendant son blocage (période off sans L-DOPA) puis après les changements cliniques pendant le déblocage (période "on" avec L-DOPA). Le temps est chronométré.

Ce test sert à :
- diagnostiquer une maladie de Parkinson,
- mesurer la sensibilité à la L-DOPA,
- établir le choix du traitement à suivre (greffe, stimulation électrique, traitement médical).

Ce test se fait avec les mesures UPDRS telles que décrites ci-avant :
- UPDRS moteur (Echelle d'Evaluation Unifiée Pour la Maladie de Parkinson) ;
   . UPDRS I (état mental du patient) ;
   . UPDRS II (vie quotidienne "on ― off") ; ADL (pour activity daily living);
   . Autoscoring test (test fait par le malade, vérification et constatation par le médecin des fluctuations des périodes "on ― off' pendant sa journée).

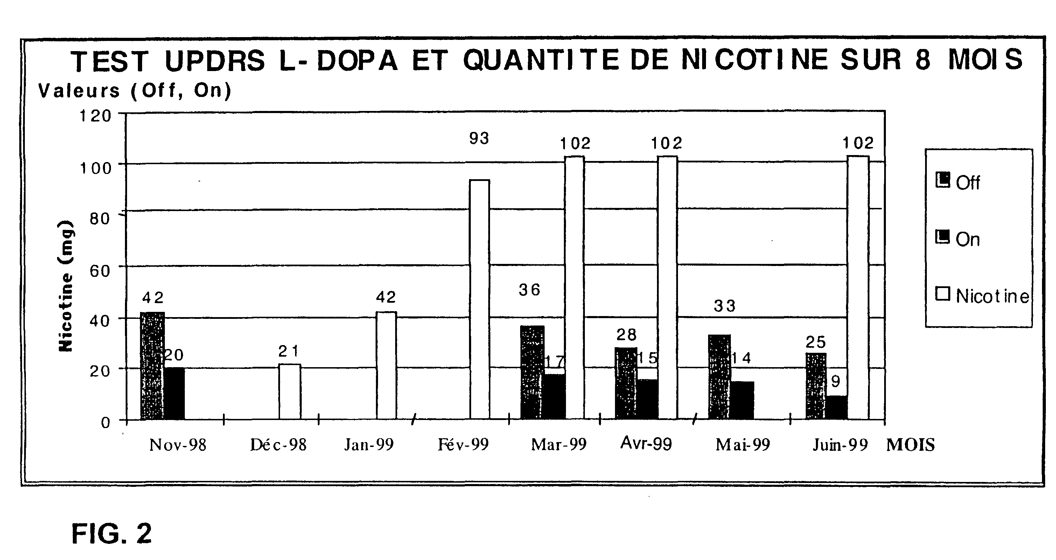

Les résultats du test "off/on" du test UPDRS pendant les périodes "off/on" sont présentés dans la figure 2. Cette figure représente les valeurs respectivement en période "off' et en période "on" en présence de 30 mg de bromocryptine, et en présence de 250 mg de L-DOPA pour le premier test "on" et 187,50 mg pour le test "on" après six mois de traitement. On peut observer une diminution du score des périodes "off' sauf au septième mois de traitement du fait de l'interruption à cette époque du traitement nicotine. En revanche, les périodes "on" font apparaître une diminution constante des mesures UPDRS. Ces mesures doivent être interprétées comme l'existence d'un effet curatif réel à long terme d'un traitement avec le médicament selon l'invention. Par ailleurs, le patient qui souffrait de troubles sexuels et de dysfonctionnement urinaire depuis deux ans a vu ces fonctions totalement rétablies après quatre mois du traitement.

De la même façon, les troubles de mémoire ont diminué ainsi que les épisodes dépressifs.

Enfin, les tableaux III et IV suivants montrent respectivement l'effet du traitement sur les manifestations physiques et sur les tests UPDRS III moteur sans et avec traitement à la nicotine.

**Tableau III:**

| **Aspect** | **Avant traitement à la nicotine** | **Avec traitement à la nicotine** | **Sans traitement à la nicotine** | **Reprise nicotine à 102 mg/kg/j** |
|---|---|---|---|---|
| Tremblement | 3 | 1 | 3 | 1 |
| Rigidité axiale | 2 | 0 | 1 | 0 |
| Marche | 2 | 0 | 1 | 0 |
| Parole | 2 | 0 | 2 | 0 |

**Tableau IV :**

| **Test** | **Sans traitement à la nicotine** **(jour 0)** | **Avec traitement à la nicotine** **(6 mois plus tard)** |
|---|---|---|
| UPDRS I (état mental) | 10 | 0 |
| UPDRS II off (activité quotidienne, ADL) | 22 | 10 |
| UPDRS II on (activité quotidienne, ADL) | 13 | 6 |

II apparaît clairement que le traitement abolit totalement la rigidité axiale, et normalise la marche et la parole (tableau III). Il y apparaît également que les manifestations neuro-psychologiques de la maladie de Parkinson disparaissent entièrement dans un délai de six mois à compter du début du traitement. La diminution des tests UPDRS II en période "off" et en période "on" y apparaît également clairement (tableau IV).

Ce traitement ne doit pas être interrompu et les doses doivent être entretenues toujours entre 93 mg et 160 mg par jour. Selon l'amélioration clinique de chaque patient, on pourrait diminuer ou augmenter la dose journalière de la nicotine (mais jamais diminuer au-dessous de 93 mg par jour). Nous pouvons utiliser la nicotine à vie selon l'évolution clinique et des symptômes des patients.

### Exemple 3 : administration de la nicotine à des doses comprises entre 0,2 et 5 mg par jour et par kilo par administration sous-cutanée :

### a) administration par une pompe extracorporelle :

Une pompe extracorporelle, de préférence portative est utilisée. Un cathéter sous-cutané permet d'administrer en permanence la dose de nicotine requise, et adaptable par un régulateur de vitesse alimenté par des piles.

### b) administration par patchs rapides et lents, éventuellement alimentés électriquement :

L'administration sous forme de patch avec ou sans pompe transcutanée doit être posée dans des endroits où la nicotine est absorbée de façon optimale et continue afin d'obtenir un taux sanguin efficace. De tels endroits sont de préférence les fesses ou les fosses lombaires.

En conclusion, si on compare les résultats obtenus avec le médicament selon l'invention, administré en synergie avec la L-DOPA à des doses sub-actives, il apparaît que, pour la première fois, on observe chez les patients un rétablissement, une diminution ou un arrêt total des syndromes caractérisant la maladie de Parkinson et des maladies associées. Les résultats des tests UPDRS I, II et III montrent en outre un rétablissement clair des fonctions dopaminergiques et nicotinergiques permettant de présumer une stabilisation à long terme de ces améliorations.

## Revendications

1. Médicament formulé pour comprendre l'administration, de façon progressive, d'une dose de 0.2 à 5 mg par jour par kilo de poids corporel chez l'homme de nicotine ou un dérivé de celle-ci, et l'administration de façon simultanée de L-DOPA à une dose comprise entre 0.2 et 3 mg par jour par kilo de poids corporel chez l'homme.

2. Médicament selon la revendication 1 comprenant en outre un agoniste dopaminergique.

3. Médicament selon la revendication 2 dans lequel l'agoniste dopaminergique est le bromocriptine ou le pyribedil.

4. Médicament selon l'une quelconque des revendications 1 à 3, selon lequel au moins un des composés est sous une forme galénique.

5. Médicament selon l'une quelconque des revendications 1 à 4, administré par voie orale, sous-cutanée, transdermique ou une combinaison de ces voies d'administration.

6. Médicament selon la revendication 5, selon lequel l'administration sous-cutanée est de préférence en patch.

7. Médicament selon l'une quelconque des revendications 1 à 6 administré durant une longue période.

8. Médicament selon la revendication 7, selon lequel ladite longue période est de quatre mois, et de préférence de six mois.

9. Médicament selon l'une quelconque des revendications 1 à 8 formulé de manière à restaurer les fonctionnalités des récepteurs dopaminergiques D1 et D2.

10. Utilisation pour administration de façon progressive, de 0,2 à 5 mg par jour et par kilo de poids corporel chez l'homme de nicotine ou un dérivé de celle-ci, ladite nicotine ou ledit dérivé de celle-ci étant administré de façon simultanée avec la L-DOPA à des doses de 0,2 mg à 3 mg par jour et par kilo de poids corporel chez l'homme, pour la fabrication d'un médicament utile pour le traitement de maladies neurodégénératives.

11. Utilisation selon la revendication 10, selon laquelle les maladies neurodégératives sont la maladie de Parkinson ou le syndrome de la Tourette.

12. Utilisation selon l'une des revendications 10 à 11, selon laquelle les doses croissantes de nicotine sont administrées de manière concomitante avec une diminution de la dose de L-DOPA.

13. Utilisation selon l'une des revendications 10 à 12, selon laquelle le médicament comprend en outre un agoniste dopaminergique.

14. Utilisation selon la revendication 13, selon laquelle l'agoniste est la bromocryptine ou le pyribédil.

15. Utilisation selon l'une des revendications 10 à 14, selon laquelle le médicament est destiné au traitement des maladies neurodégénératives pendant une durée supérieure à quatre mois.

16. Utilisation selon la revendication 15, selon laquelle le médicament est destiné au traitement des maladies neurodégénératives pendant une période de six mois.

17. Utilisation selon l'une des revendications 10 à 16, selon laquelle le médicament est administré par voie transdermique, sous-cutanée, orale ou une combinaison de ces voies d'administration.

18. Utilisation selon l'une quelconque des revendications précédentes, selon laquelle au moins un des composés du médicament est sous forme galénique.

19. Utilisation selon l'une des revendications 17 ou 18, selon laquelle le médicament est sous forme d'un patch transdermique.

20. Utilisation selon l'une des revendications 17 ou 18, selon laquelle le médicament est sous forme liquide et administré par voie sous-cutanée à l'aide d'une pompe extracorporelle.

## Patentansprüche

1. Medikament, welches so formuliert ist, daß es die progressive Verabreichung einer Dosis von 0,2 bis 5 mg Nikotin oder eines Derivats davon pro Tag und Kilogramm Körpergewicht an einen Menschen und die gleichzeitige Verabreichung von L-DOPA in einer Dosis zwischen 0,2 und 3 mg pro Tag und Körpergewicht an einen Menschen umfaßt.

2. Medikament nach Anspruch 1, welches weiter einen Dopamin-Agonisten umfaßt.

3. Medikament nach Anspruch 2, wobei der Dopamin-Agonist Bromcryptin oder Pyribedil ist.

4. Medikament nach einem der Ansprüche 1 bis 3, wobei mindestens eine der Verbindungen in galenischer Form vorliegt.

5. Medikament nach einem der Ansprüche 1 bis 4, welches auf oralem, subkutanem, transdermalem Weg oder einer Kombination dieser Verabreichungswege verabreicht wird.

6. Medikament nach Anspruch 5, wobei die transdermale Verabreichung vorzugsweise in Form eines Pflasters erfolgt.

7. Medikament nach einem der Ansprüche 1 bis 6, welches über einen langen Zeitraum hinweg verabreicht wird.

8. Medikament nach Anspruch 7, wobei der lange Zeitraum 4 Monate, vorzugsweise 6 Monate, beträgt.

9. Medikament nach einem der Ansprüche 1 bis 8, welches so formuliert ist, daß es die Funktionalitäten der Dopamin D1- und D2-Rezeptoren wiederherstellt.

10. Verwendung zur progressiven Verabreichung von 0,2 bis 5 mg Nikotin oder eines Derivats davon pro Tag und Kilogramm Körpergewicht an einen Menschen, wobei das Nikotin oder das Derivat davon gleichzeitig mit L-DOPA in Dosen von 0,2 mg bis 3 mg pro Tag und Kilogramm Körpergewicht an einen Menschen verabreicht werden, zur Herstellung eines Medikaments, das zur Behandlung von neurodegenerativen Erkrankungen von Nutzen ist.

11. Verwendung nach Anspruch 10, wobei die neurodegenerativen Erkrankungen Parkinson-Krankheit oder Tourette-Syndrom sind.

12. Verwendung nach einem der Ansprüche 10 bis 11, wobei die ansteigenden Dosen an Nikotin bei damit einhergehender Verringerung der Dosis an L-DOPA verabreicht werden.

13. Verwendung nach einem der Ansprüche 10 bis 12, wobei das Medikament weiter einen Dopamin-Agonisten umfaßt.

14. Verwendung nach Anspruch 13, wobei der Agonist Bromcryptin oder Pyribedil ist.

15. Verwendung nach einem der Ansprüche 10 bis 14, wobei das Medikament zur Behandlung neurodegenerativer Erkrankungen über einen Zeitraum von mehr als vier Monaten bestimmt ist.

16. Verwendung nach Anspruch 15, wobei das Medikament zur Behandlung neurodegenerativer Erkrankungen über einen Zeitraum von sechs Monaten bestimmt ist.

17. Verwendung nach einem der Ansprüche 10 bis 16, wobei das Medikament auf transdermalem, subkutanem, oralem Wege oder einer Kombination dieser Verabreichungswege verabreicht wird.

18. Verwendung nach einem der vorhergehenden Ansprüche, wobei mindestens eine der Verbindungen des Medikaments in galenischer Form vorliegt.

19. Verwendung nach einem der Ansprüche 17 oder 18, wobei das Medikament in Form eines transdermalen Pflasters vorliegt.

20. Verwendung nach einem der Ansprüche 17 oder 18, wobei das Medikament in flüssiger Form vorliegt und auf subkutanem Wege mit Hilfe einer außerhalb des Körpers befindlichen Pumpe verabreicht wird.

## Claims

1. Medicament formulated for administration in a progressive manner at a dose of 0.2 to 5 mg per day per kilogram body weight in a human of nicotine or a derivative thereof, and for administration in a simultaneous manner with L-DOPA at a dose between 0.2 and 3 mg per day by kilogram body weight in a human.

2. Medicament according to claim 1, further comprising a dopaminergic agonist.

3. Medicament according to claim 2, wherein the dopaminergic agonist is bromocriptine or pyribedil.

4. Medicament according to any one of claims 1 to 3, wherein at least one of the compositions is in a galenical form.

5. Medicament according to any one of claims 1 to 4, which is administered through the oral route, the sub-cutaneous route, the transdermic route or a combination of these administrative routes.

6. Medicament according to claim 5, wherein the sub-cutaneous administration is preferably the patch.

7. Medicament according to any one of claims 1 to 6, which is administered over a long period.

8. Medicament according to claim 7, wherein the long period is for four months and preferably for six months.

9. Medicament according to any one of claims 1 to 8, formulated in a manner to restore the functionality of D1 and D2 dopaminergic receptors.

10. Use of nicotine or a derivative thereof for administration in a progressive manner at a dose of 0.2 to 5 mg per day per kilogram body weight in a human of nicotine or a derivative thereof, and for administration in a simultaneous manner with L-DOPA at a dose between 0.2 and 3 mg per day by kilogram body weight in a human for the fabrication of a medicament to treat neurodegenerative diseases.

11. Use according to claim 10, wherein the neurodegenerative disease are Parkinson's disease or Tourette's syndrome.

12. Use according to any one of claims 10 to 11, wherein increasing doses of nicotine are administered simultaneously with a decreasing dose of L-DOPA.

13. Use according to any one of claims 10 to 12, wherein the medicament further comprises a dopaminergic agonist.

14. Use according to claim 13, wherein the antagonist is bromocryptine or pyribedil.

15. Use according to any one of claims 10 to 14, wherein the medicament is destined to treat neurodegenerative diseases during duration greater than four months.

16. Use according to claim 15, wherein the medicament is destined to treat neurodegenerative diseases during duration of six months.

17. Use according to any of claims 10 to 16, which is administered through the oral route, the sub-cutaneous route, the transdermic route or a combination of these administrative routes.

18. Use according to any one of the preceding claims, wherein at least one of the compositions is in a galenical form.

19. Use according to one of the claim 17 or 18, wherein the medicament is in the form of a transdermal patch.

20. Use according to one of the claims 17 or 18, wherein the medicament is in liquid form and is administered subcutaneously by an extracorporal pump.
